# EUROPEAN PATENT APPLICATION

(11) **EP 4 751 692 A1**
(43) Date of publication of application: **03.06.2026**
(21) Application number: 24216964.7
(22) Date of filing: 02.12.2024
(51) Int. Cl.: A61F 13/513, A61F 13/514

(54) **ABSORBENT ARTICLE WITH BARRIER CUFFS AND ZONE TREATED TOPSHEET**

(71) Applicant: Ontex BV, 9255 Buggenhout (BE); Ontex Group NV, 9320 Erembodegem (BE)
(72) Inventor: SCHULTE, Lea, 56727 Mayen (DE); PSCHYKLENK, Lukas, 56761 Müllenbach (DE); Reuter, Agnes Dominika, 53359 Rheinbach (DE); Lambertz, Christina, 56566 Neuwied (DE); Sprotte, Katharina, 56073 Koblenz (DE); Pannwitt, Stefanie, 56727 Mayen (DE); FOCHLER, Alisa, 56727 Mayen (DE)
(74) Representative: Macchetta, Andrea

(57) **Abstract**

An absorbent article comprising a chassis including a liquid-pervious topsheet, a backsheet, and a liquid absorbent core disposed between the liquid-pervious topsheet and the backsheet, the chassis having a front region, a back region, a crotch region located between the front region and the back region, a longitudinal axis extending through the front and back regions, a lateral axis substantially perpendicular to the longitudinal axis, a first longitudinal side edge and a second longitudinal side edge, wherein the topsheet has a maximum width measured along the lateral axis where the topsheet has its greatest width and wherein the backsheet comprises a liquid impervious film layer and an outer cover layer, said absorbent article further comprising a pair of barrier cuffs extending in a longitudinal direction from the front region to the back region along the topsheet, each barrier cuff comprising an inwards unattached portion and an outwards attached portion with a hinge point therebetween, wherein the topsheet comprises one or more first zones and at least two second zones wherein the first zone(s) is substantially hydrophilic and wherein the second zones are hydrophobic; and wherein the attached portion of each of said barrier cuffs overlaps at least one of the second zones and does not substantially overlap the first zone(s); and/or wherein the attached portion of each of said barrier cuffs is positioned proximal or adjacent to a second zone and distal from said first zone(s) such that the attached portion is separated from said first zone(s) by said second zone.

## Description

### TECHNICAL FIELD

The present disclosure pertains to the technical field of absorbent hygiene articles such as disposable diapers or (diaper) pants, disposable incontinence diapers or pants, pads, provided with barrier cuffs reducing the risk of side leakage and offering comfort. Preferably the disclosure relates to disposable pants.

### BACKGROUND

Absorbent articles for personal hygiene are designed to absorb and contain bodily exudates, such as a large quantity of urine. Side leakage is a common problem for absorbent articles. Gushes of urine discharge can temporarily flood the surface of the absorbent article, leaking over the side margin.

To adress this problem, elastic gathering elements have been incorporated in various ways along the lateral edges of the absorbent article. These may include barrier cuffs which are elasticized flaps which stand substantially upright, more preferably inwardly towards the longitudinal centerline of the article, at least within the crotch region. Typically, said barrier cuffs include at least one elastic that is connected primarily at its opposing ends to the diaper. When the absorbent article is worn, typically, the uprising barrier cuffs extend through the groin area and along both of the buttocks of the wearer. The elastic gathering elements may also include gasketing cuffs, also called leg elastics, which are elasticized flaps which do not stand substantially upright, and which are more preferably disposed outwardly towards the longitudinal side edges of the article, within the crotch region. Typically, said gasketing cuffs include at least one elastic that is connected substantially throughout its length to the diaper (e.g., multiple bonds along length of elastic to create gathers). When the absorbent article is worn, typically, the gasketing cuffs will encircle the thighs and create a gasketing action against the thighs.

WO2007/043027, for example, describes a barrier cuff strip extending in a longitudinal direction from the front waist region to the back waist region along the topsheet. The barrier cuff is shown as being formed by securing a separate element to the topsheet between the gasketing cuffs and the side edge of the absorbent core. The proximal edge of the barrier cuff is formed by securing the barrier cuff element to the topsheet by attachment means and the distal edge of the barrier cuff is spaced away from the topsheet top surface by the elastic gathering action of the spacing elastic members.

Although barrier cuffs are today very efficient in reducing the risk of side leakage, we have noted that in certain circumstances, liquid could still pass through this barrier and cause leakage towards the longitudinal sides of absorbing articles.

The present disclosure aims to provide a novel absorbent article particularly designed to even more reduce the risks of side leakage, whilst not compromising on other aspects like absorbency and comfort.

### SUMMARY

In one aspect, the present disclosure relates to an absorbent article comprising a chassis including a liquid-pervious topsheet, a backsheet preferably joined to the topsheet, and a liquid absorbent core disposed between the liquid-pervious topsheet and the backsheet, the chassis having a front region, a back region, a crotch region located between the front region and the back region, a longitudinal axis extending through the front and back regions, a lateral axis substantially perpendicular to the longitudinal axis, a first longitudinal side edge and a second longitudinal side edge, wherein the topsheet has a maximum width measured along the lateral axis where the topsheet has its greatest width and wherein the backsheet comprises a liquid impervious film layer and an outer cover layer, said absorbent article further comprising a pair of barrier cuffs extending in a longitudinal direction from the front region to the back region along the topsheet, each barrier cuff comprising an inwards unattached portion and an outwards attached portion with a hinge point therebetween, wherein the topsheet comprises one or more first zones and at least two second zones wherein the first zone(s) is substantially hydrophilic and wherein the second zones are hydrophobic; and wherein the attached portion of each of said barrier cuffs overlaps at least one of the second zones and does not substantially overlap the first zone(s); and/or wherein the attached portion of each of said barrier cuffs is positioned proximal or adjacent to a second zone and distal from said first zone(s) such that the attached portion is separated from said first zone(s) by said second zone (typically in a width direction extending substantially parallel to the lateral axis)..

We have found that the circumstances under which side leakage could still occur could somehow be linked to the nature of the topsheet used in the absorbent article, for example with fully hydrophilic and/or more premium lofty topsheets, soft topsheets, air-through bonded topsheets, topsheets based on natural materials and/or topsheets in relief, e.g. with 3D structures, i.e. embossed topsheets. We have further found that the liquid could actually be guided via the topsheet, under the cuff outwards attached portion, towards the sides of the article. Without wishing to be bound by theory, we believe that topsheets have a more open structure which allows the liquid to exit under the cuff outwards attached portion by capillarity. We have then found that zone treatment in combination with the constructions herein was a solution to reduce this side leakage problem. One way of making this possible is by using a zoned treated topsheet arranged in combination with cuff positioning as described herein. Alternatively or in addition, by ensuring that the maximum width of the topsheet is less than or equal to a distance measured along the lateral axis between the hinge points of each barrier cuff and that the barrier cuffs are free of any attachment to the topsheet in the crotch region. This reduction of the risk of side leakage may be even more advantageous for absorbent articles of higher sizes, where bigger amount of urine is to be handled and/or the wearer moves more. A further advantage associated with the use of a narrower topsheet according to embodiments herein may lead to a cost reduction and/or CO2 reduction as less material is used.

Other objects and advantages of this disclosure will become apparent hereinafter.

### BRIEF DESCRIPTION OF THE FIGURES

FIG. 1 is a schematized top plan view of an absorbent article according to embodiments herein, in a flat-out, uncontracted state (i.e., without elastic induced contraction) with the portion of the article which contacts the wearer facing the viewer.
FIG. 2 is a schematized exploded transverse sectional view of the chassis of FIG. 1, as viewed along X-X, according to an embodiment herein.
FIG. 3a to 3E are schematized exploded transverse sectional view of the chassis of FIG. 1, as viewed along X-X, according to embodiments herein.
Fig. 4 shows a side view of the body-shaped test equipment and associated suspended belt as used in the example tests.
FIG. 5 is a top plan view of an absorbent article according to embodiments herein, in a flat-out, uncontracted state (i.e., without elastic induced contraction) with the portion of the article which contacts the wearer facing the viewer.

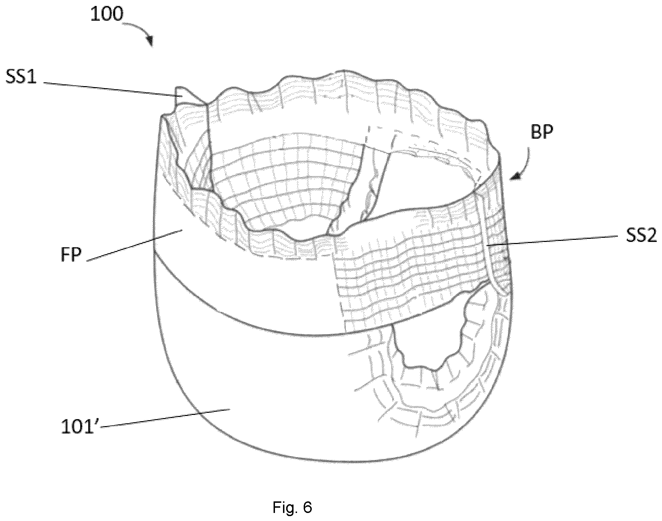

FIG. 6 is an isometric view of an absorbent article according to embodiments herein, such as that of Fig. 5 in assembled state.
FIG. 7 is a top plan view of a topsheet comprising zone treatment according to embodiments herein.
FIG. 8 is a top plan view of a topsheet comprising zone treatment and an absorbent core according to an embodiment herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners, Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. The absorbent article includes a chassis. The absorbent article may include liquid wicking layers, liquid intake layers, liquid distribution layers, transfer layers, barrier layers, wrapping layers and the like, as well as combinations thereof. Disposable absorbent articles and the components thereof can operate to provide a skin-facing surface and a garment-facing surface.

A "chassis" of a disposable absorbent articles can include a liquid pervious topsheet, a backsheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The chassis comprises a front waist portion or region, a back waist portion or region, an intermediate crotch portion or region which interconnects the front and back waist portions. When used herein, reference to a "front" portion refers to that part of the chassis which is generally located on the front of a wearer when in use. Reference to the "back" portion refers to the portion of the chassis generally located at the back of the wearer when in use, and reference to the "crotch" portion refers to that portion which is generally located between the legs of a wearer when in use. The crotch portion is an area where repeated fluid surge typically occurs.

The "absorbent core" is the absorbent structure disposed between the topsheet and the backsheet of the chassis and is capable of absorbing and retaining liquid body exudates. The size and the absorbent capacity of the absorbent core should be compatible with the size of the intended wearer and the liquid loading imparted by the intended use of the chassis. Further, the size and the absorbent capacity of the absorbent core can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent core. It can contain embossed channels, channels substantially free of material, channels with lower basis weight than the rest of the core, etc. The absorbent core is disposed between the topsheet and the backsheet; both the topsheet and the backsheet generally extend beyond the side edges of the absorbent core. The juxtaposed areas of the topsheet and the backsheet are typically secured together such as by adhesive.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help to prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

"Bonded" refers to the joining, adhering, connecting, attaching, or the like, of at least two elements. Two elements will be considered to be bonded together when they are bonded directly to one another or indirectly to one another, such as when each is directly bonded to intermediate elements. "Join", "joining", "joined", or variations thereof and "attach", "attaching", "attached", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined or attached together either directly or indirectly, i.e. they may have one or more elements interposed between them, all of which are connected together. For the sake of clarity, adhesive is not to be considered as an intervening element for the definition of direct or indirect attachment. When describing an attachment "without intervening layer of material" or "without any intervening material", this does not exclude the presence of adhesive.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which two elements are directly or indirectly joined. For example configurations where top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The term "backsheet" refers to a material forming a liquid impervious cover of the chassis of the absorbent article. The back sheet prevents the exudates contained in the absorbent core from wetting articles such as bedsheets and overgarments which contact the disposable absorbent article. The back sheet may be a composite layer composed of multiple components assembled side-by-side or laminated. The back sheet may be the same or different in different parts of the chassis. At least in the area of the absorbent core, the back sheet comprises a liquid impervious material in the form of a thin plastic film, e.g., a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials, in such laminates, the polymeric film can have the same or a different width than the nonwoven material. The term "breathable" refers to films having a water vapor transmission rate (WVTR) of at least 300 grams/m² - 24 hours.

As used herein, the "skin-facing" or "bodyside" or " body-facing" or "user facing" or "inner" side or surface means that surface of the article or component which is intended to be disposed toward or placed adjacent to the body of the wearer during ordinary use, while the "outward", "outward-facing", "garment-facing" or "garment" or "outer" side or surface is on the opposite side, and is intended to be disposed to face away from the wearer's body during ordinary use. Such outward surface may be arranged to face toward or placed adjacent to the wearer's undergarments when the absorbent article is worn.

Further, an absorbent article can comprise "standing gathers" or "containment flaps" or "barrier cuffs". The standing gathers are generally thought to be particularly well suited for the containment of fecal matter and to prevent the lateral flow of liquid waste until such time as the liquid waste can be absorbed by the absorbent article. Many constructions of standing gather are known. Such standing gather generally comprises a proximal portion intended to be attached to the chassis, and an opposed distal portion which is generally not attached to the chassis along at least a portion of its length. An elastic member is generally located on or adjacent a distal edge to provide elasticity to the standing gather, to assist in maintaining the containment flap in an upright condition and in maintaining a sealing relationship between the distal edge of the standing gather and the body of a wearer during use. The elastic member is generally located between two layers of material so that the elastic does not come into contact with the body of a wearer. The standing gather may be manufactured from any of a wide variety of materials such as woven, nonwoven, spunbonded, carded, cast, blown or the like, or combinations or laminations thereof. The materials may include any of a variety of compositions including but not limited to polypropylene, polyester, rayon, nylon, foams, plastic films, formed films, and elastic foams. To improve containment, the material may be hydrophobic, or at least partially liquid impervious. A number of manufacturing techniques may be used to manufacture the containment flaps.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The term "flat configuration" or "laid-flat state" or "fully stretched state" or "extended state" or "flat-out" configuration is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "topsheet" refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven. Suitable nonwoven materials can be composed of man-made fibres, such as polyester, polyethylene, polypropylene, viscose, rayon etc. or natural fibers, such as wood pulp or cotton fibres, hemp, and the like, or other natural or naturally-derived fibers, or from a mixture of two or more natural or man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g., urine or menstrual fluid. The inner coversheet may further be different in different parts of the chassis of an absorbent article. The top sheet fabrics may be composed of a substantially hydrophobic material, and the hydrophobic material may optionally be treated with a surfactant or otherwise processed to impart a desired level of wettability and hydrophilicity.

The term "nonwoven" or "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes. Nonwovens may be embossed via an embossing process whereby a pattern is pressed into the fabric, usually by passing the material between rolls with little clearance, and where one or both rolls has a raised design. "Three dimensional embossed nonwovens", also called "embossed nonwovens" may provide enhanced softness and cushioning.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used herein, the term "transverse" or "lateral" refers to a line, axis, or direction which lies within the plane of the absorbent article and is generally perpendicular to the longitudinal direction (i.e., is parallel to the X-X axis).

The term "bio-based" may be used herein to refer to a component of an absorbent article that can be produced or is derived from a renewable resource. The term "renewable resource" is used herein to refer to a natural resource that may be produced by a natural process at a rate comparable to its rate of consumption (e.g., within a 100 year time frame). The resource may be replenished naturally, or via engineered agricultural techniques. Nonlimiting examples of renewable resources may include plants, animals, fish, bacteria, fungi, and forestry products. These resources may be naturally occurring, hybrids, or genetically engineered organisms. Natural resources such as crude oil, coal, and peat that take longer than 100 years to form are generally not considered to be renewable resources.

"Natural fibers" refers to elongated substances produced by plants and animals and includes animal-based fibers and plant-based fibers, as those categories are described herein. Natural fibers, as that term is used herein, include fibers harvested without any post-harvest treatment step, as well as those having a post-treatment step, such as, for example, washing, scouring, bleaching, etc.

"Plant-based fibers," as that term is used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut.

"Macro-perforations" or "openings" as used herein means perforations/openings that are several magnitudes greater than the pore size (generally formed between fibers) of the materials referred to e.g. nonwovens. Several suitable techniques may be employed to make such perforations/openings such as needle-punching, thermos-pressure embossing, appropriately patterned screens in combination with hydroentanglement of fibers via water jets and the like.

Embodiments according to the disclosure will now be described. It is understood that technical features described in one or more embodiments may be combined with one or more other embodiments without departing from the intention of the disclosure and without generalization therefrom.

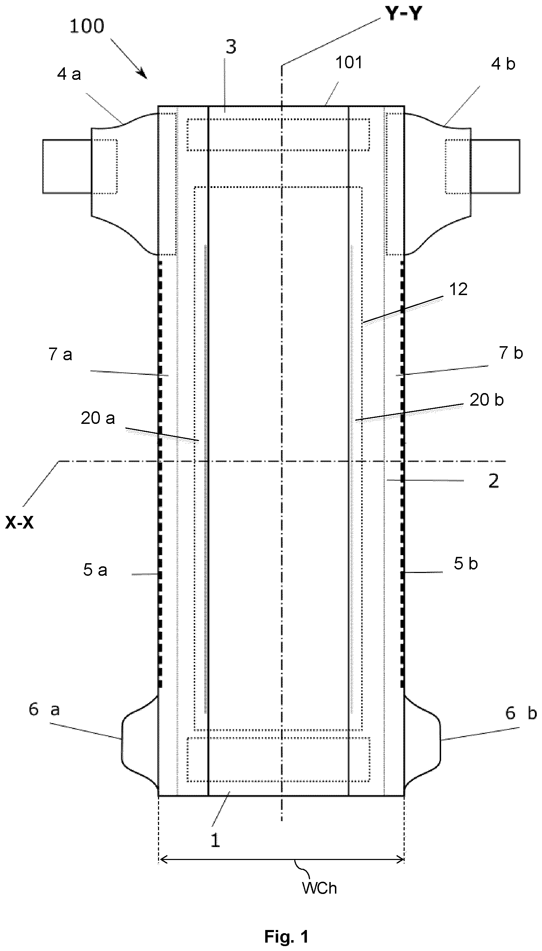

Absorbent articles 100 according to the present disclosure, as for example illustrated in Fig. 1, 5 and Fig.2, comprise a chassis 101 (herein also referred to as "insert", especially for embodiments where the article is a pant, see for example chassis/insert 101') including a liquid-pervious topsheet 10, typically defining at least a portion of the user-facing or skin-facing side of the chassis, a backsheet 15, typically defining at least a portion of the garment-facing side of the chassis, said backsheet preferably being joined to the topsheet 10, and a liquid absorbent core 12 disposed between the liquid-pervious topsheet 10 and the backsheet 15. The backsheet 15 comprises a liquid impervious film layer 13 and an outer cover layer 14.

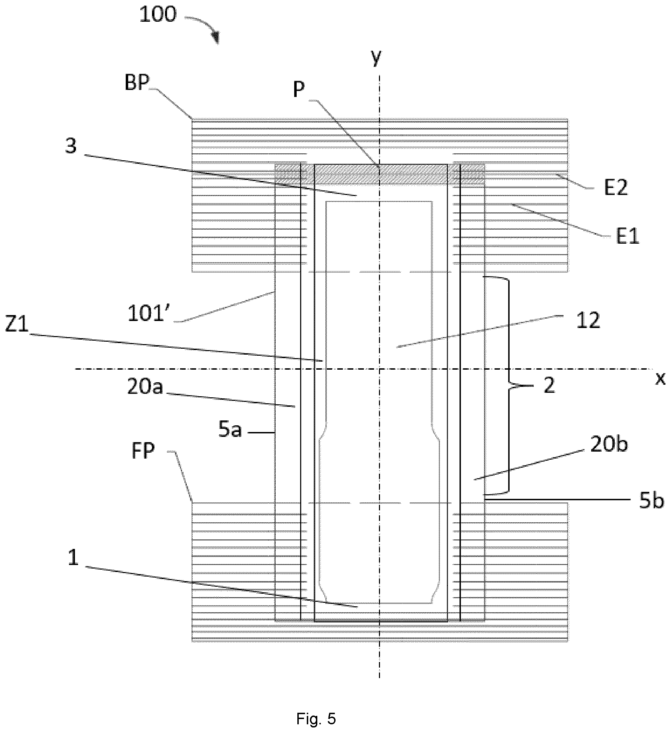

The chassis 101, as for example illustrated in Fig. 1, has a front waist region 1 (especially in pant embodiments herein such as illustrated in Fig. 5 also referred to as "front region"), a back waist region 3 (especially in pant embodiments herein such as illustrated in Fig. 5 also referred to as "back region"), an intermediate crotch region 2, which interconnects the front and back waist regions, and a periphery which is defined by the outer edges of the chassis in which the first and second longitudinal side edges are respectively designated 5a and 5b and the front and back transverse edges are respectively designated 8a and 8b. The chassis additionally has a lateral axis X-X which is here drawn as a centerline and a longitudinal axis Y-Y which is here drawn as a centerline. The longitudinal axis Y-Y extends through the front and back waist regions, and the lateral axis X-X is substantially perpendicular to the longitudinal axis. While the chassis 101, 101' is illustrated in FIG. 1 and Fig. 5 as having a generally rectangular shape, it may have other shapes or contours, e.g. an hourglass shape, as desired.

As typical in absorbent articles, the chassis 101, 101' comprises the main structure of the article with other features added to form the composite diaper or pant structure. For example, the diaper may include separate "ears" or side panels attached to the chassis 101 at the back waist region 3, called back side panels 4a, 4b. In some embodiments, front side panels 6a, 6b may be attached to the chassis 101 at the front waist region 1, for example, instead of or in addition to at the back waist region 3. In some embodiments, such back side panels 4a, 4b and/or front side panels 6a, 6b could be integral with the chassis or be separately attached. For example, as illustrated in Fig. 5 ad 6, the pant may comprise front and back belts (or panels) FP, BP. These belts/panels are generally adapted to come into contact with the waist of the user and are preferably joined to each other through at least two opposite side seams SS1, SS2 that may be permanent or refastenable. The belts/panels preferably comprising a plurality of first elastics E1 and/or one or more second elastics E2, preferably wherein the elastic spacing between first elastics is different, more preferably greater than, the elastic spacing between second elastics.

The articles herein may further comprise one or more transversal cuffs and/or pockets (P) at the front and/or back of said article. Such pockets may be as for example described on paragraphs 0072 and 0073 of EP4454624A1 to which cross reference is made herein.

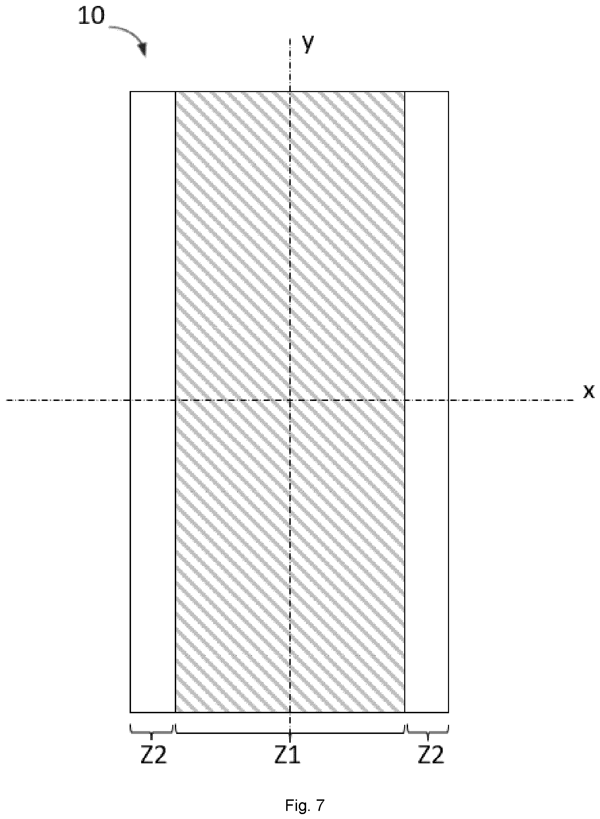

As exemplarily illustrated in Fig.7, the topsheet (10) herein comprises one or more first zones (Z1) and at least two second zones (Z2) wherein the first zone(s) (Z1) is substantially hydrophilic [typically meaning that at least semi-durable hydrophilic properties are imparted as explained in more detail in embodiments herein] and wherein the second zones (Z2) are hydrophobic; and in that the attached portion (23) of each of said barrier cuffs overlaps at least one of the second zones (Z2) and does not substantially overlap [typically meaning that minor overlaps, e.g. due to production tolerances, are encompassed], preferably does not overlap, the first zone(s) (Z1); and/or in that the attached portion (23) of each of said barrier cuffs is positioned proximal or adjacent to a second zone (Z2) and distal from said first zone(s) (Z1) such that the attached portion (23) is separated from said first zone(s) (Z1) by said second zone (Z2) preferably in a width direction extending substantially parallel to the lateral axis X-X. Advantageously this arrangement allows to significantly reduce the risk of leakage that could otherwise arise from liquid permeating through the hydrophilic portions of the topsheet under the cuffs.

Nonwovens made from synthetic fibers, such as polyolefins like polypropylene, polyethylene and the like, are generally hydrophobic in nature. By application of suitable finishing treatments (also referred to as spinfinish), it is possible to impart semi-durable hydrophilic properties to the nonwoven to achieve performance in liquid strike-through and liquid runoff that is required for their use in absorbent products. Suitable finishing treatments are typically proprietary blends of synthetic surfactant solutions which are commercially available, for example, from Schill & Seilacher AG (e.g. Silastol PHP 26, Silastol PHP 90, & Silastol 163), and Pulcra Chemicals (e.g. Stantex S 6327, Stantex S 6087-4, & Stantex PP 602). Traditionally they are typically applied to the nonwovens full surface in the range of 0.004-0.006 gm solids/gm nonwoven (i.e. 0.4-0.6% wt/wt). An example of a synthetic surfactant that has been widely used in commercially-available topsheet finishes would be Triton GR-5M, an anionic sulfosuccinate surfactant manufactured by Dow Chemical Company. Other types of surfactants used are based on fatty acid polyethylene glycol esters. Any such spinfinish solutions, and the like, are suitable herein as long as applied according to the zones (or zone treatment) described herein.

The second zone(s) (Z1) is preferably substantially interposed between at least two of the said second zones (Z2), said at least two of the said second zones (Z2) extending longitudinally along the first and second longitudinal edges of the chassis (101, 101'). The hydrophobic zones arranged in this manner may form retarding barriers to liquid.

It is preferred that said article is a pant comprising discrete front and back panels (FP, BP) wherein said chassis (101') is joined to each of said front and back panels (FP, BP) so that said panels are indirectly connected to each other by said chassis, and preferably directly connected to each other at a pair of side seams (SS1, SS2) to form a waist opening and two leg openings.

Preferably, the first zone(s) (Z1) is located centrally of the topsheet (10) and the second zones are located outboard of the first zone(s) (Z1) and are positioned along at least a portion of a perimeter of the topsheet (10) preferably at least comprising longitudinal edges running substantially parallel to the first and second longitudinal side edges (5a, 5b) of the chassis (101'). Advantageously this arrangement enhances the formation of retarding barriers to liquid and work synergistically with the cuffs as arranged as described herein.

The topsheet may comprise, preferably consists of, synthetic fibers that are generally hydrophobic, and wherein the first zone(s) (Z1) is hydrophilically treated with a substance, said substance preferably comprising one or more surfactants.

Preferably, the topsheet has a first area and wherein the first zone(s) (Z1) has a second area and wherein the area ratio of second area/first area is from 0.65 to less than 1, preferably from more than 0.65 to 0.95, even more preferably from 0.68 to 0.90, even more preferably from 0.70 to 0.89, even more preferably from 0.73 to 0.85, even more preferably from 0.75 to 0.80. Advantageously such ratios allow for maximised surface area useful for liquid absorption and core effectiveness whilst at the same time maximising liquid barrier effects laterally in a crotch region of the article and hence minimize leakages.

The first zone(s) (Z1) and the second zones (Z2) are preferably elongate and extend along a length running along the longitudinal axis (Y-Y) of the topsheet and/or article; and preferably wherein the first zone(s) and/or second zones are in the form of stripes. Said first zone(s) (Z1) may extend along less than 100% of said length of the topsheet, preferably from more than 60% to less than 95%, even more preferably from 70% to 90%, of said length. Advantageously this arrangement further limits risk of leakage at transversal terminal edges of the article (these generally being the edges that connect to the longitudinal side edges 5a,5b to form the perimeter of the chassis 101', and that extend substantially parallel to the axis X-X).

The first zone(s) (Z1) may have a total width of more than 110mm, preferably more than 120mm, preferably more than 125mm, even more preferably from 128mm to 160mm. Advantageously such sizing allows for maximised fluid permeability for core effectiveness yet without having to increase the overall width of the topsheet and hence limiting cots and waste.

Each cuff (20a, 20b) may be joined to at least a portion of the topsheet at the attached portion (23). However, when this is done it is preferred to ensure that at least the majority of the attachment overlaps the hydrophobic zones as described herein.

The topsheet may be a nonwoven having basis weight of from 10gsm to 35gsm, preferably comprising, more preferably consisting of, polyolefin fibers. Preferably, the topsheet is a nonwoven selected from spunbond nonwovens, carded nonwovens, and combinations thereof; and wherein the topsheet comprises a plurality of macro-perforations or openings. Advantageously this may further improve management of liquid stool and limit leakage thereof.

Preferably, the first zone(s) (Z1) has a width in a direction parallel to the lateral axis (X-X) that is greater or equal to a width of the absorbent core (12), preferably being substantially equal to the width of the absorbent core (12). Advantageously this ensures maximised liquid permeability to the core for absorption and core utilisation.

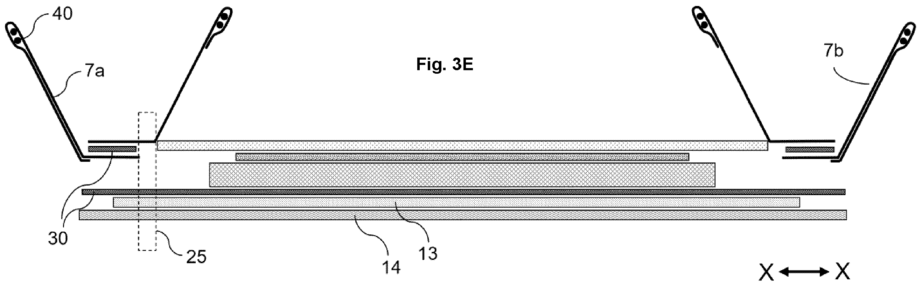

In preferred embodiments, the article may include gasketing cuffs 7a, 7b, also called leg elastics, which are elasticized flaps more preferably disposed outwardly towards the longitudinal side edges of the article, within the crotch region. Typically, said gasketing cuffs include at least one elastic member 40 that is connected substantially throughout its length to the diaper. The at least one elastic member 40 generally extends at least along the crotch region and may also extend in at least part of the front and/or back waist regions of the chassis. When the absorbent article is worn, typically, the gasketing cuffs will encircle the thighs and create a gasketing action against the thighs. The gasketing cuffs may be integral with the chassis (as for example illustrated in Figures 1, 2, 3 A-D) or be separately attached thereto (as for example illustrated in Figure 3 E).

Absorbent articles 100 according to the present disclosure comprise a pair of barrier cuffs 20a, 20b extending in a longitudinal direction from the front waist region to the back waist region along the topsheet 10. Each barrier cuff 20a, 20b comprises an inwards unattached portion 21 and an outwards attached portion 23 with a hinge point 22a, 22b therebetween. Preferably, said barrier cuffs 20a, 20b include at least one elastic member 24 that is connected primarily at its opposing ends to the diaper. By contraction of said elastic member, the unattached portion of the barrier cuffs typically assumes an upright configuration in at least the crotch region of the diaper to form a seal against the wearer's body. When the absorbent article is worn, typically, the uprising unattached portion of the barrier cuffs extend through the groin area and along both of the buttocks of the wearer.

In an embodiment, the maximum width of the topsheet WT, i.e. the width of the topsheet measured along the lateral axis X-X where the topsheet 10 has its greatest width, is less than or equal to a distance DH measured along the lateral axis X-X between the hinge points 22a, 22b of each barrier cuff 20a, 20b. In this embodiment, the each of the attachment portions of the cuffs are positioned proximal and/or adjacent the second zones (Z2) preferably such that there is no overlap of the said attachment portions and the said second zones (Z2). Advantageously this permitting excellent leakage prevention with the formation of a multi-phase barrier being a system designed to provide layered protection against liquid leakage by incorporating multiple, distinct stages of defense. Each "phase" in the barrier system serves a specific function, working together to enhance overall performance as will be described in more detail herein. In some embodiments, the difference between the maximum width of the topsheet WT and the distance DH measured along the lateral axis X-X between the hinge points 22a, 22b is at most 6 mm or at most 5 mm, preferably at most 4 mm or at most 3 mm, more preferably at most 2 mm or 1 mm. Most preferably the difference between the maximum width of the topsheet WT and the distance DH measured along the lateral axis X-X between the hinge points 22a, 22b is 0 mm. An advantage of ensuring that such distance is small or even nil may be that the liquid impervious film layer, which is generally made of PE, comes as less as possible, or not even, in contact with the wearer's skin, therefore reducing the risk of discomfort of having plastic touching the skin. In addition, when the liquid impervious film layer is printed with graphics on its user facing face, this may reduce the risk of having inks in contact with the skin.

In an embodiment, the barrier cuffs 20a, 20b are free of any attachment to the topsheet 10 in the crotch region 2 and may be directly joined to the liquid impervious film layer 13 typically of the backsheet.

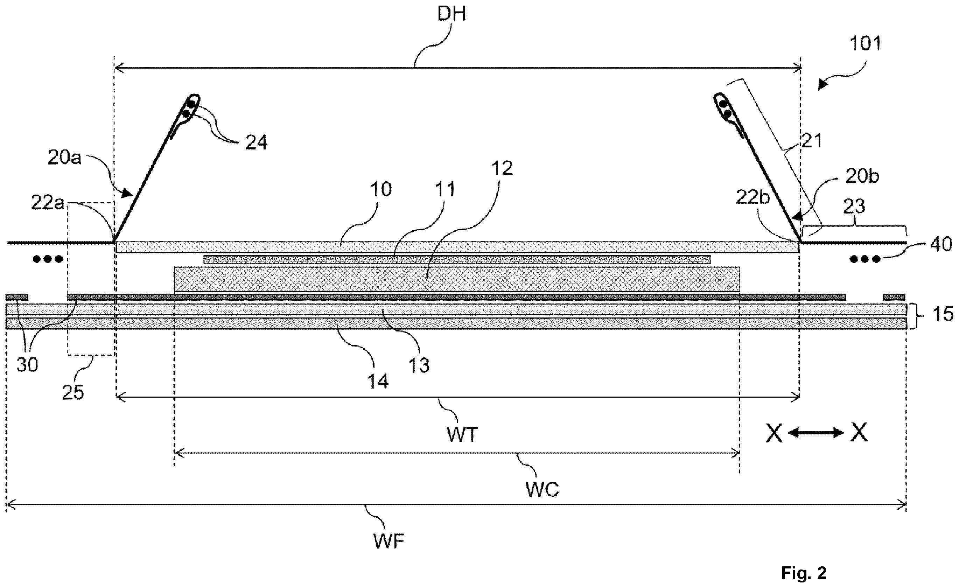

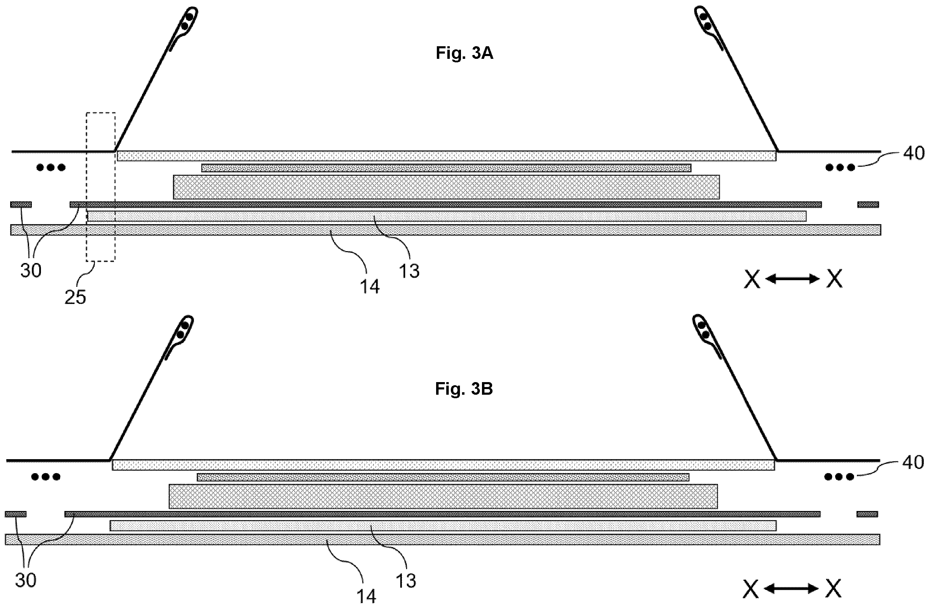

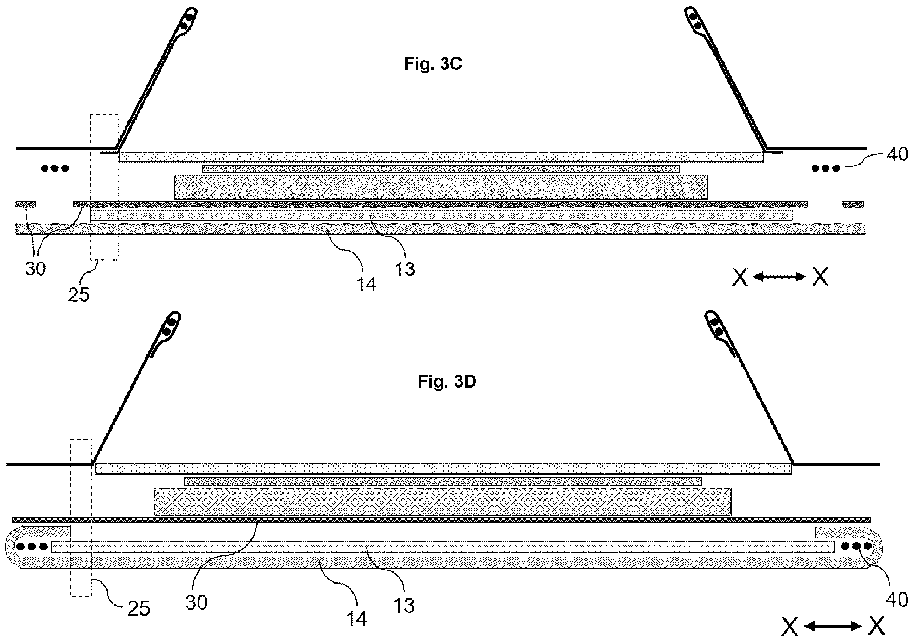

In a preferred embodiment, the barrier cuffs 20a, 20b, and in particular the outwards attached portion 23 of the barrier cuffs 20a, 20b, may be attached in many different configurations, optionally as long as they are free of any attachment to the topsheet 10 in the crotch region 2. These configurations include attachment to the liquid impervious film layer 13, to the outer cover layer 14, or to both the liquid impervious film layer 13 and the outer cover layer 14, with or without intervening layer of material, i.e. respectively directly and indirectly. They may for example be attached (i) directly to the liquid impervious film layer 13, as for example in Fig. 2, (ii) directly to the liquid impervious film layer 13 and the outer cover layer 14, as for example in Fig. 3A, 3C, 3D when the liquid impervious film layer 13 is narrower than the outer cover layer, (iii) to the outer cover layer 14, as for example in Fig. 3B when the liquid impervious film layer 13 has the same width as the liquid-pervious topsheet 10, or (iv) to the liquid impervious film layer 13 and the outer cover layer 14 with intervening separately attached gasketing cuffs 7a, 7b, as for example in Fig. 3E. These are all configurations which minimise the risk of side leakage according to the present disclosure.

Advantageously, a part 25 of the outwards attached portion 23 of the barrier cuffs 20a, 20b closest to the hinge point 22a, 22b may be directly, i.e. without any intervening material, attached to the liquid impervious film layer (13) of the backsheet (15), as illustrated in Figures 2, 3A and 3C to 3E. This may further improve side leakage reduction by ensuring an improved attachment. For example gluing of the outwards attached portion of the barrier cuffs to a film generally made of PE may improve lamination strength compared to typically gluing to a nonwoven.

The outwards attached portion 23 of the barrier cuffs 20a, 20b may be attached by various bonding methods, such as with adhesive, mechanical bonding, thermal bonding, pressure bonding, ultrasonic bonding, combinations of these, or the like. Exemplary figures herein all show adhesive bonds 30. The bonding may extend over the full surface of attachment or may be present in stripes, in dots or any adequate pattern ensuring appropriate bonding.

In an embodiment, the outer cover layer 14 of the backsheet 15 can be folded back on itself along the longitudinal side edges 5a, 5b, either towards the user facing side of the article (as illustrated in present Fig. 3D) or towards the garment facing side of the article, and encase at least one elastic member 40, thereby forming gasketing cuffs 7a, 7b. This is described in International Patent Application N° PCT/EP2023/086716, in particular from page 14 line 1 to page 15 line 31, together with Figures 1B to 1E. Alternatively, a wrapper element encasing the at least one elastic member 40 in a folded portion may be joined to the backsheet along the longitudinal side edges 5a, 5b, as described in International Patent Application N° PCT/EP2023/086716, in particular from page 15 line 33 to page 17 line 24, together with Figures 1F to 1J.

In an embodiment, absorbent articles according to the present disclosure may be further characterised by the liquid absorbent core 12 having a maximum width WC measured along the lateral axis X-X where the core has its greatest width, or by the liquid impervious film layer 13 of the backsheet 15 having a minimum width (WF) measured along the lateral axis (X-X) where the film layer has its smallest width, or by the chassis 101 having a minimum width (WCh) measured along the lateral axis (X-X) from the first longitudinal side edge (5a) to the second longitudinal side edge (5b) where the chassis has its smallest width, such that the article shows at least one or more of the following dimensions:
i. a ratio WT/WC between the maximum width of the topsheet and the maximum width of the core is comprised between 1.15 and 1.50, preferably between 1.15 and 1.38, more preferably between 1.20 and 1.37, even more preferably between 1.22 and 1.36;
ii. a ratio WT/WF between the maximum width of the topsheet and the minimum width of the backsheet film layer is comprised between 0.65 and 0.89, preferably between 0.65 and 0.85, more preferably between 0.68 and 0.82, even more preferably between 0.70 and 0.80;
iii. a ratio WT/WCh between the maximum width of the topsheet and the minimum width of the chassis is comprised between 0.60 and 0.77, preferably between 0.62 and 0.75, more preferably between 0.65 and 0.73.

These ranges show particularly good side leakage protection.

The barrier cuffs 20a, 20b may be manufactured from a wide variety of materials such as polypropylene, polyester, rayon, nylon, foams, plastic films, formed films, and elastic films. The barrier cuffs may be provided with absorbent means and/or may be rendered liquid impermeable. A number of manufacturing techniques may be used to manufacture the barrier cuff. For example, the barrier cuff may be woven, nonwoven, spunbonded, carded, or the like. Barrier cuffs may comprise a substrate such as a nonwoven or a nonwoven-film laminate. Examples of such substrate are the 15 gsm HO Super soft SMS sold by Fitesa Sweden AB under reference SC6KW-30 15NN or the 15 gsm PEGATEX SMS Softbond hydrophobic sold by PFN.

The barrier cuff inwards unattached portion 21 may have a width measured along the lateral axis X-X of at least 2 mm, preferably between 5 and 50 mm, more preferably between 25 and 40 mm. These ranges provide an effective barrier to side leakage.

The distal edge of the barrier cuff inwards unattached portion 21 is preferably formed by folding an end of the material back upon itself. This provides comfort as this is coming into contact with user skin. Elastic members 24 may advantageously be enclosed in a tunnel that is formed when the end of the inwards unattached portion 21 is folded back on itself. As illustrated in the Figures, this fold back may have different lengths, shorter as in Fig. 2, 3A, 3B, 3D, 3E, to save on material, or longer as in Fig. 3C to provide a double layer cuff which may provide an enhanced leakage protection.

The elastic member 24 is preferably secured to the barrier cuffs 20a, 20b in an elastically contractible condition so that in a normally unrestrained configuration, the elastic member 24 effectively contracts or gathers the barrier cuffs. The length of the elastic member 24 in general is dictated by the absorbent article design. In the embodiment illustrated in FIG. 1, the elastic member 24 extends essentially the entire length of the barrier cuffs 20a, 20b in the crotch region 2, although other lengths are possible.

The absorbent article preferably additionally includes a bond and/or multiple bonds, such as an adhesive glue bead, for securing closed the front and back ends of each barrier cuff 20a, 20b. The areas in which the bond(s) are disposed are generally designated front and back tack-down regions. In such regions, the inwards unattached portion 21 of the barrier cuffs 20a , 20b is secured to the topsheet top surface by a bond such as a glue bead. This bond could also be accomplished through a variety of mechanisms such as adhesive, mechanical bonding, thermal bonding, pressure bonding, ultrasonic bonding, or the like. Therefore, in these areas, the distal edge of the inwards unattached portion 21 is generally closed, i.e. it is not spaced away from the topsheet top surface. It may be that the elastic member 24 is not disposed in this region because the distal edge is not designed to be spaced away from the topsheet top surface in the waist regions.

Elastic members 24, 40 may be operatively associated with their respective cuff by securing them within said cuff with an elastic attachment element. The elastic attachment element should be flexible and of sufficient adhesiveness to hold elastic members in their stretched condition. Elastic members, having a first and second end, may be secured to their respective cuff only near their ends or along their entire length. Elastic attachment element may be glue beads made of hot melt adhesive. Alternatively, elastic attachment element may take the form of an ultrasonic bond or heat/pressure seal. One or more elastic members may be used in each cuff. Elastic members which have been found suitable are elastic strands having a cross section of 0.18 mm by 1.5 mm, or elastic tapes having a cross section of about 0.18 mm by 0.52 mm, made from natural rubber. Elastic members may also comprise any heat shrinkable elastic material as is well known in the art. Other suitable elastic materials may comprise a wide variety of materials as are well known in the art including elastomeric films, polyurethane films, elastomeric foams, formed elastic scrim and synthetic elastomers (e.g., LYCRA). In addition, elastic members 24, 40 may take a multitude of configurations. For example, the width may be varied; a single strand or several parallel or non-parallel strands of elastic material may be used; or a variety of shapes may be used including rectilinear and curvilinear. Elastic members 24, 40 are preferably sheets, strands or ribbons of natural rubber, synthetic rubber, thermoplastic elastomeric polymers or any other elasticized material known in the art.

Elastic members 40 may be attached to the gasketing cuffs 7a, 7b in a stretched condition, while the gasketing cuffs are in a gathered state, or joined to the gasketing cuffs and then elasticized or shrunk, for example with the application of heat, so that elastic retractive forces are imparted to the gasketing cuffs. The at least one elastic member 40 of the the gasketing cuffs 7a, 7b, may extend over the crotch region 2 and at least into part of the front waist region 1 and back waist region 3.

A suitable topsheet 10 may be manufactured from a wide range of materials, such as apertured plastic films, woven materials, nonwoven materials, woven or nonwoven materials of natural fibers (e.g., wood or cotton fibers), synthetic fibers or filaments (e.g., a polyester or a polyolefin, such as polyethylene (PE), polypropylene (PP), or bicomponent PE/PP fibers or mixtures thereof), or a combination of natural and synthetic fibers. According to the present disclosure, the topsheet 10 may be a nonwoven material having one or multiple formed layers, nonwovens, or components comprising plant-based fibers and/or fibers comprising a recycled content of for example more than 50%, preferably other than wood pulp. The nonwoven may be made through well-known techniques; for example, the nonwoven may be a spunbond, a carded and air-through, hydroentangled, or calendar bonded nonwoven. The plant-based fibers may also be spun fibers made at least in part from bio-based materials. For example, the plant-based fibers may be spun poly lactic acid (PLA) fibers or bio-based polyolefin spun fibers. The plant-based fibers may be single component fibers or multi-component fibers, in which less than all of the components are plant-based fibers. One example is a bi-component fiber comprising a polyester core component and a bio-based polyethylene sheath component.

The topsheet 10 may have one or more layers. The topsheet may be apertured, may have any suitable three-dimensional features, and/or may have a plurality of embossments. The topsheet may have hydrophilic and/or hydrophobic portions or layers. The topsheet 26 may have a basis weight of from about 10 gsm to about 40 gsm.

The present disclosure is particularly advantageous with, but not limited to, topsheet materials which may have at least one of the following characteristics: bulky, lofty, soft, with visual embossing, with perforations, air-through bonded, spunbond, spunmelt, calendered, highloft, semi-highloft, and/or which may comprise micro-fibers, PE, PET, and/or bio-based polymers as for example PLA. In some embodiments, the topsheet 10 is selected from air-through bonded nonwovens, embossed nonwovens and nonwovens comprising plant-based fibers. An exemplary topsheet is a spunbond nonwoven from PFN, 100 Wt% PP. In some embodiments, the liquid-pervious topsheet 10 consists of a single nonwoven layer or consists of a nonwoven underlayer on which an upper nonwoven patch is attached, said patch being shorter than the nonwoven underlayer in both width along the lateral axis X-X and length along the longitudinal axis Y-Y. The use of such a patch may provide loftiness, bulkiness, premium sensation and/or embossing in a dedicated zone where it is most wanted, while saving on material.

Chassis 101, 101' may further include an acquisition and distribution layer 11 between the core and the topsheet and/or between the core and the backsheet.

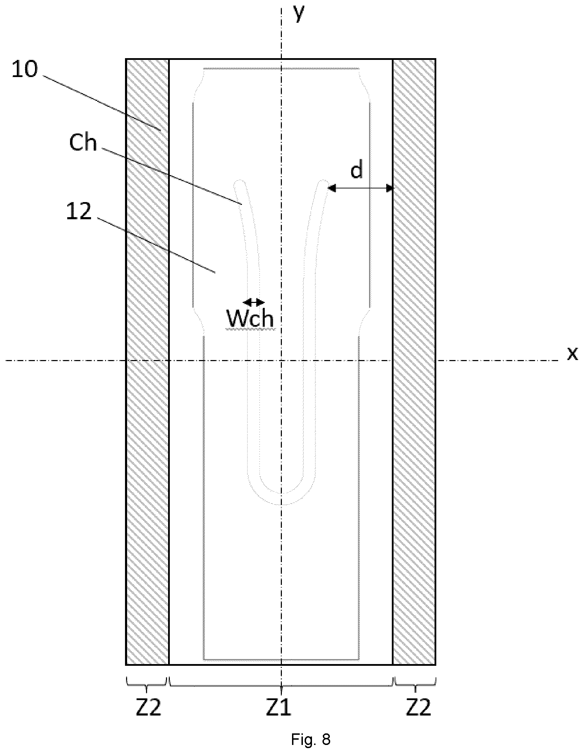

In a particularly preferred embodiment, as for example illustrated in Fig. 8, the absorbent core (12) comprises a core wrap substantially enclosing absorbent material therein and wherein top and bottom layers of the core wrap are joined together to form one or more channels (Ch) substantially free of absorbent material and preferably being circumscribed by absorbent material, and wherein the one or more channels overlap the first zone(s) (Z1) and preferably do not overlap the second zones (Z2). Advantageously, channels improve liquid distribution and allow for better core utilization and it has been found that when using a zone treated topsheet as described herein it is desirable to ensure such channels overlap the hydrophilic zone (Z1) and preferably don't overlap the hydrophobic zone (Z2). The hydrophilic zone overlapping the channel allows for faster core in-flow, at the same time hydrophobic zone overlapping the channel increases risks of moisture being collected in a respective portion thereof that may cause skin irritation over time and this being exacerbated by the presence of a channel in such zone that would be guiding more liquid thereto and hence preferably avoided.

Although Fig.8 illustrates a U-shaped channel, any suitable channel shape may be used herein including multiple distinct channels of the same or different shapes and/or comprising straight portions and/or curved portions and the like.

Preferably, the absorbent article comprises one or more channels (Ch) having a width (Wch) and positioned at a distance from the second zones (Z2) such that said one or more channels does not overlap, or is not adjacent to, the second zones (Z2) . Advantageously this allows for reduced risk of skin irritation and/or leakage for similar reasons described above with the formation of a unique multi-phase barrier when in conjunction with cuffs arranged as described herein.

Preferably, a distance (d) between the one or more channels and the closest of the second zones (Z2) is at least 1.5 times the channel width **Wch,** preferably from 1.6**Wch** to 10.5**Wch,** even more preferably from 1.9**Wch** to 9.5**Wch,** even more preferably from 2.5**Wch** to 8.5**Wch,** even more preferably from 3**Wch** to 8**Wch,** even more preferably from 3.5**Wch** to 7.5**Wch,** even more preferably from 4**Wch** to 7**Wch.** Preferably, said distance (d) is the shortest distance between the channel and the closest second zone (Z2) thereto generally along an axis being substantially parallel to the x-axis.(also referred to herein as lateral axis). Advantageously, this arrangement allows to further reduce the risk of leakage and/or skin irritation.

Absorbent articles as herein described may take the form of an open diaper or a pant for babies, children or adults. They may include a re-usable outer shell that is coupled with chassis 101, which acts as a disposable absorbent "insert".

### BODY-SHAPED TEST METHOD

Equipment needed:
- Body-shaped test equipment
- Marker
- Ruler
- Metal Board
- Magnets
- Beaker
- Laboratory balance (scale graduation 0,01g)
- Measuring cylinder
- 2 x Electronic clock (accuracy 1s per 20 min)
- Filter paper (for quantitative analysis, fast, round filter 90 mm, e.g. Schleicher & Schuell type 589/1, black band or type 604. Others must show comparable results)
- Weights: to be selected according to the size of the tested diaper. See Table I.

**Table I :**

| Diaper size | Weights in kg |
|---|---|
| Mini | 2,5 |
| Midi | 4,5 |
| Maxi | 10,5 |
| Maxi plus | 13,0 |
| Junior | 16,0 |

- NaCl solution (0,9%) made by dissolving 9 g sodium chloride, NaCl p.a. in 991 g demineralised water. Only chemicals with p.a.-quality have to be used. The water should be demineralised (conductivity < 5 µS/cm) or have the adequate pureness. A food colorant free of common salt was added to the solution.

### Body-shaped test equipment

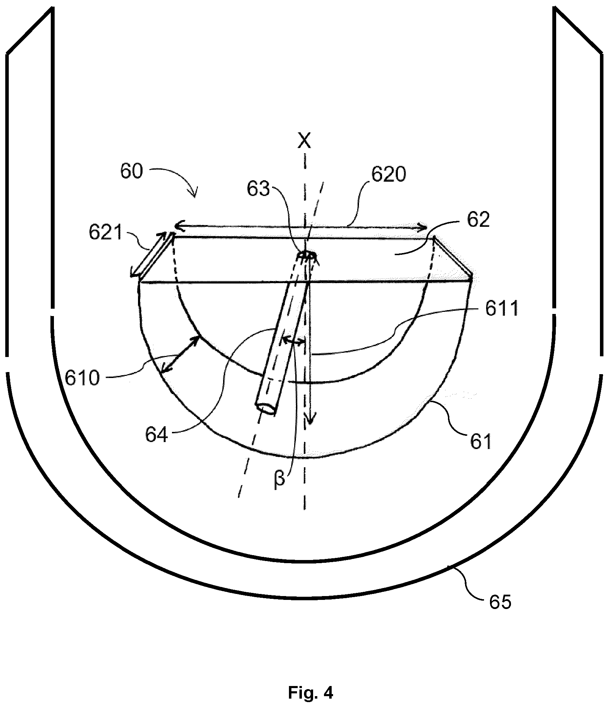

The body-shaped test equipment is a bended apparatus (60) as illustrated in Figure 4, used to place a diaper and maintain it in cup shaped position.

This apparatus (60) comprises an arc-shaped transparent plate (61) along which a diaper can be placed and maintained in position, e.g. with clamps, with its full surface against the external, underneath face of the plate. The width (610) of the arc-shaped plate is 21,5 cm. The apparatus (60) also comprises a horizontal rectangular transparent plate (62) which has a length (620) of 36 cm and a width (621) of 21,5 cm, and which joins the two sides of the arc-shaped plate at a height (611) taken along the axis of symmetry (X) of the arc shape equal to 20 cm. This horizontal plate (62) comprises a hole (63) in its center. The apparatus (60) further comprises a tube (64) of 30 mm diameter and of 22 cm length. It is inclined with an angle β of about 14° from verticality (X).

### Preparation of samples

This test has to be performed on at least 3 products. The diapers are laid flat by fixing them on a metal board. A transversal centreline is drawn, dividing the diaper into two equal parts.

The diaper is placed onto the body-shaped test equipment (60) against the external, underneath face of the arc-shaped plate (61), at room temperature, so that the marked centreline on the diaper is crossing the axis of symmetry (X) of the arc shape, with the front region of the diaper on the side where the tube (64) comes in proximity with the diaper, i.e. the liquid intake point. Barrier cuffs are lying flat on the diaper.

The body-shaped test equipment together with the diaper are then placed within a suspended belt (65) having a U shape and having a similar width than the width (610) of the arc-shaped plate (61). The belt (65) maintains the diaper in contact with the arc-shaped plate (61) and holds the body-shaped test equipment in the air. The belt may be made out of a canvas cover, for example made of PVC.

### Test procedure

The test equipment is subjected to a load depending on the weight category of the tested diaper (see Table I). Weights are placed on horizontal plate (62).

Then 0,9% NaCl solution is weighted into measuring cylinder, depending on the weight category of the tested diaper (see Table II):

**Table II:**

| Diaper size | Amount of liquid in g |
|---|---|
| Mini | 40 |
| Midi | 60 |
| Maxi | 80 |
| Maxi plus | 85 |
| Junior | 100 |

A first amount of liquid according to Table II is added to the test equipment through opening (63). At the same time the stopwatch and timer are started and set to 10 minutes. After 10 minutes a second amount of liquid according to Table II is added again.

The liquid distribution to the sides is reported after all the liquid vanished from the tube (64). The result, given in mm, is the maximum liquid extension towards the longitudinal side edges, i.e. the greatest width reached by the liquid, as measured along the lateral axis (X-X) of the diaper with the ruler. The test is repeated on 3 articles, so that the final result is the average of the results obtained on the 3 diapers.

### EXAMPLE AND COMPARATIVE EXAMPLE

Tests have been performed on 3 diapers according to the invention, and 3 others not in accordance with the invention, to monitor and compare the distribution of a saline solution in a baby diaper using the herein described body-shaped test method, thereby showing some of the benefits of the invention.

### Example 1

3 identical diapers as illustrated in Fig. 1 and Fig. 3A, in size Junior, have been receiving 2 shots of 100 g liquid at an interval of 10 minutes, as described in the hereinabove body-shaped test method.

The diapers were characterised by an absorbent core having a U-shaped channel as described in WO2018122117A1. The barrier cuffs were free of any attachment to the topsheet in the crotch region. They were attached directly to the liquid impervious film layer in a zone closest to the hinge point and directly to the outer cover layer in proximity to the longitudinal side edges, as the liquid impervious film layer was narrower than the outer cover layer. The topsheet was an air-trough bonded nonwoven comprising 85 Wt% PE/PET and 15% cotton commercialized under the name ParaTherm Loft 533/22 by TWE.

### Comparative Example 1

3 diapers identical to those of Example 1, except that the topsheet was broader and that the barrier cuffs were attached directly to the topsheet in the crotch region in a zone closest to the hinge point, have been subjected to the same test.

| | Example 1 | Comparative Example 1 |
|---|---|---|
| maximum width of the topsheet: WT | 145 mm | 177 mm |
| distance measured along the lateral axis between the hinge points of each barrier cuff : DH | 145 mm | 145 mm |
| WT - DH | 0 mm | 32 mm |
| maximum width of the core : WC | 120 mm | 120 mm |
| WT/WC | 1.21 | 1.48 |
| minimum width of the backsheet film layer : WF | 200 mm | 200 mm |
| WT/WF | 0.73 | 0.89 |
| minimum width of the chassis : WCh | 220 mm | 220 mm |

Liquid distribution to the sides after 2 shots was measured with the following results:

| | Example 1 | Comparative Example 1 |
|---|---|---|
| diaper 1 | 150 mm | 175 mm |
| diaper 2 | 145 mm | 180 mm |
| diaper 3 | 145 mm | 175 mm |
| average | 146.67 mm | 176.67 mm |

These results clearly show the advantage of example 1 according to the present invention, where the liquid barely extends outwards of the hinge points of each barrier cuff, whilst it distributes under the cuff outwards attached portion, towards the longitudinal sides in comparative example 1. In this comparative example, the barrier cuff does not act as a barrier to maintain the liquid in the central portion of the diaper where the core is present and can absorb the liquid. The liquid wets the longitudinal sides of the diaper in contact with the user's skin which may induce discomfort and rash, and liquid leaks to the side may occur.

### Example 2

3 diapers identical to those of Example 1, except for the topsheet have been subjected to the same test. In example 2, the topsheet was a plain (i.e. not embossed) spunbond comprising 100 Wt% polypropylene, commercialized under the name PEGATEX S - soft 18 g/m² - white - hydrophilic with Aloe Vera and Vitamin E, by PFN Nonwovens.

### Comparative Example 2

3 diapers identical to those of Example 2, except that the topsheet was broader and that the barrier cuffs were attached directly to the topsheet in the crotch region in a zone closest to the hinge point, have been subjected to the same test.

| | Example 2 | Comparative Example 2 |
|---|---|---|
| maximum width of the topsheet: WT | 145 mm | 175 mm |
| distance measured along the lateral axis between the hinge points of each barrier cuff : DH | 145 mm | 145 mm |
| WT - DH | 0 mm | 30 mm |
| maximum width of the core : WC | 120 mm | 120 mm |
| WT/WC | 1.21 | 1.46 |
| minimum width of the backsheet film layer : WF | 190 mm | 190 mm |
| WT/WF | 0.76 | 0.92 |
| minimum width of the chassis : WCh | 220 mm | 220 mm |

Liquid distribution to the sides after 2 shots was measured with the following results:

| | Example 2 | Comparative Example 2 |
|---|---|---|
| diaper 1 | 145 mm | 160 mm |
| diaper 2 | 140 mm | 150 mm |
| diaper 3 | 145 mm | 160 mm |
| average | 143.33 mm | 156.67 mm |

These results show again the advantage of the invention where the liquid does not extend outwards of the hinge points of each barrier cuff in example 2, whilst it distributes under the cuff outwards attached portion, towards the longitudinal sides in comparative example 2, and this even if the topsheet is rather plain and basic and not particularly lofty or embossed. Whilst these particular examples relate to diapers, the same findings are observed with pants / diaper pants.

It is assumed that the present disclosure is not restricted to any form of embodiment described above and that some modifications can be made to the presented embodiments of the invention without departing from the scope of the embodiments as set forth in the claims that follow.

## Claims

1. An absorbent article (100) comprising a chassis (101, 101') including a liquid-pervious topsheet (10), a backsheet (15), and a liquid absorbent core (12) disposed between the liquid-pervious topsheet (10) and the backsheet (15), the chassis having a front region (1), a back region (3), a crotch region (2) located between the front region (1) and the back region (3), a longitudinal axis (Y-Y) extending through the front and back regions, a lateral axis (X-X) substantially perpendicular to the longitudinal axis, a first longitudinal side edge (5a) and a second longitudinal side edge (5b), wherein the topsheet (10) has a maximum width (WT) measured along the lateral axis (X-X) where the topsheet (10) has its greatest width and wherein the backsheet (15) comprises a liquid impervious film layer (13) and an outer cover layer (14), said absorbent article further comprising a pair of barrier cuffs (20a, 20b) extending in a longitudinal direction from the front region to the back region along the topsheet (10), each barrier cuff (20a, 20b) comprising an inwards unattached portion (21) and an outwards attached portion (23) with a hinge point (22a, 22b) therebetween, **characterised in that** the topsheet (10) comprises one or more first zones (Z1) and at least two second zones (Z2) wherein the first zone(s) (Z1) is substantially hydrophilic and wherein the second zones (Z2) are hydrophobic; and **in that** the attached portion (23) of each of said barrier cuffs overlaps at least one of the second zones (Z2) and does not substantially overlap the first zone(s) (Z1); and/or **in that** the attached portion (23) of each of said barrier cuffs is positioned proximal or adjacent to a second zone (Z2) and distal from said first zone(s) (Z1) such that the attached portion (23) is separated from said first zone(s) (Z1) by said second zone (Z2).

2. An absorbent article according to claim 1, wherein the second zone(s) (Z1) is substantially interposed between at least two of the said second zones (Z2), said at least two of the said second zones (Z2) extending substantially along the first and second longitudinal edges of the chassis (101, 101').

3. An absorbent article according to any of the preceding claims, wherein said article is a pant comprising discrete front and back panels (FP, BP) wherein said chassis (101') is joined to each of said front and back panels (FP, BP) so that said panels are indirectly connected to each other by said chassis, and preferably directly connected to each other at a pair of side seams (SS1, SS2) to form a waist opening and two leg openings.

4. An absorbent article according to any of the preceding claims, wherein the first zone(s) (Z1) is located centrally of the topsheet (10) and the second zones are located outboard of the first zone(s) (Z1) and are positioned along at least a portion of a perimeter of the topsheet (10) preferably at least comprising longitudinal edges running substantially parallel to the first and second longitudinal side edges (5a, 5b) of the chassis (101').

5. An absorbent article according to any of the preceding claims, wherein the topsheet comprises, preferably consists of, synthetic fibers that are generally hydrophobic, and wherein the first zone(s) (Z1) is hydrophilically treated with a substance, said substance preferably comprising one or more surfactants.

6. An absorbent article according to any of the preceding claims, wherein the topsheet has a first area and wherein the first zone(s) (Z1) has a second area and wherein the area ratio of second area/first area is from 0.63 to less than 1, preferably from more than 0.65 to less than 1, more preferably from more than 0.65 to 0.95, even more preferably from 0.68 to 0.90, even more preferably from 0.70 to 0.89, even more preferably from 0.73 to 0.85, even more preferably from 0.75 to 0.80.

7. An absorbent article according to any of the preceding claims, wherein the first zone(s) (Z1) and the second zones (Z2) are elongate and extend along a length running along the longitudinal axis (Y-Y) of the topsheet and/or article; and preferably wherein the first zone(s) and/or second zones are in the form of stripes.

8. An absorbent article according to any of the preceding claims, wherein the first zone(s) (Z1) has a total width of more than 110mm, preferably more than 120mm, preferably more than 125mm, even more preferably from 128mm to 160mm.

9. An absorbent article according to any of the preceding claims, wherein each cuff (20a, 20b) is joined to at least a portion of the topsheet at the attached portion (23).

10. An absorbent article according to any of the preceding claims, wherein the topsheet is a nonwoven having basis weight of from 10gsm to 35gsm, preferably comprising, more preferably consisting of, polyolefin fibers.

11. An absorbent article according to any of the preceding claims, wherein the topsheet is a nonwoven selected from spunbond nonwovens, carded nonwovens, and combinations thereof; and wherein the topsheet comprises a plurality of macro-perforations or openings.

12. An absorbent article according to any of the preceding claims, wherein the first zone(s) (Z1) has a width in a direction parallel to the lateral axis (X-X) that is greater or equal to a width of the absorbent core (12), preferably being substantially equal to the width of the absorbent core (12).

13. An absorbent article according to any of the preceding claims, wherein the maximum width of the topsheet (WT) is less than or equal to a distance (DH) measured along the lateral axis (X-X) between the hinge points (22a, 22b) of each barrier cuff (20a, 20b) and in that the barrier cuffs (20a, 20b) are substantially free of any attachment to the topsheet (10) in the crotch region (2); and preferably wherein the difference between the maximum width of the topsheet (WT) and the distance (DH) measured along the lateral axis (X-X) between the hinge points (22a, 22b) is at most 6 mm, preferably at most 4 mm, more preferably at most 2 mm, most preferably 0 mm.

14. An absorbent article according to any of the preceding claims, wherein the outwards attached portion (23) of the barrier cuffs (20a, 20b) is attached to the liquid impervious film layer (13), to the outer cover layer (14), or to both the liquid impervious film layer (13) and the outer cover layer (14), with or without intervening layer of material.

15. An absorbent article according to any of the preceding claims, wherein a part (25) of the outwards attached portion (23) of each barrier cuff (20a, 20b) closest to the hinge point (22a, 22b) is directly attached to the liquid impervious film layer (13) of the backsheet (15).

16. An absorbent article according to any of the preceding claims, wherein the liquid absorbent core (12) has a maximum width (WC) measured along the lateral axis (X-X) where the core has its greatest width, wherein the liquid impervious film layer (13) of the backsheet (15) has a minimum width (WF) measured along the lateral axis (X-X) where the film layer has its smallest width and wherein the chassis (101) has a minimum width (WCh) measured along the lateral axis (X-X) from the first longitudinal side edge (5a) to the second longitudinal side edge (5b) where the chassis has its smallest width, **characterised in that** the article shows at least one or more of the following dimensions:
i. a ratio WT/WC between the maximum width of the topsheet and the maximum width of the core is comprised between 1.15 and 1.50, preferably between 1.15 and 1.38, more preferably between 1.20 and 1.37, even more preferably between 1.22 and 1.36;
ii. a ratio WT/WF between the maximum width of the topsheet and the minimum width of the backsheet film layer is comprised between 0.65 and 0.89, preferably between 0.65 and 0.85, more preferably between 0.68 and 0.82, even more preferably between 0.70 and 0.80;
iii. a ratio WT/WCh between the maximum width of the topsheet and the minimum width of the chassis is comprised between 0.60 and 0.77, preferably between 0.62 and 0.75, more preferably between 0.65 and 0.73.

17. An absorbent article according to any of the preceding claims, wherein the absorbent core (12) comprises a core wrap substantially enclosing absorbent material therein and wherein top and bottom layers of the core wrap are joined together to form one or more channels (Ch) substantially free of absorbent material and preferably being circumscribed by absorbent material, and wherein the one or more channels overlap the first zone(s) (Z1) and preferably do not overlap the second zones (Z2).

18. An absorbent article according to any of the preceding claims, wherein the absorbent article comprises one or more channels (Ch) having a width (Wch) and positioned at a distance from the second zones (Z2) such that said one or more channels does not overlap, or is not adjacent to, the second zones (Z2) .

19. An absorbent article according to any of he preceding claims, wherein a distance (d) between one or more channels (Ch) and the second zone (Z2) closest thereto is at least 1.5 times the channel width Wch, preferably from 1.6Wch to 10.5Wch, even more preferably from 1.9Wch to 9.5Wch, even more preferably from 2.5Wch to 8.5Wch, even more preferably from 3Wch to 8Wch, even more preferably from 3.5Wch to 7.5Wch, even more preferably from 4Wch to 7Wch.
